# EUROPEAN PATENT APPLICATION

(11) **EP 1 623 815 A1**
(43) Date of publication of application: **08.02.2006**
(21) Application number: 04018457.4
(22) Date of filing: 04.08.2004
(51) Int. Cl.: B29C 65/02, A61B 5/022

(54) **Producing method of an air bag for inflating**

(71) Applicant: TK Incorporated Company, Tokyo (JP)
(72) Inventor: Kawamura, Takao, Tokyo (JP)
(74) Representative: Bergen, Klaus

(57) **Abstract**

The method of the invention includes a step of jointing, in a sheet (1) formed in a rectangular shape, end portions (4,5) of a pair of mutually opposed two sides in such a manner as to form a jointed portion of a predetermined width along a planar direction of the sheet (1). In the rectangular sheet (1), a pair of mutually opposed two end portions (4,5) are jointed by a joint portion formed along the planar direction of the sheet.

## Description

### BACKGROUND OF THE INVENTION

The present invention relates to an improvement on a method for producing an air bag.

A sphygmomanometer is generally equipped with an air bag constituting a cuff to be wound on for pressurizing an arm of an inspected person at a blood pressure measurement.

Such air bag, for use as a cuff (arm band) of a sphygmomanometer, has been produced by superposing two rectangular resin sheets and sealing the entire periphery for example by welding.

In the prior air bag for cuff produced in such manner, the air blown therein at the use of the sphygmomanometer is compressed to constantly exert a pressure acting from the interior of the bag to the exterior, so that the sealed joint in the entire periphery of the aforementioned resin sheets is constantly subjected to a stress for peeling the sealed joint, and, in case the welded bonding in such sealed joint is weaker in a portion than in other positions, a peeling may result in the use over years to cause air leakage from such portion, whereby the sphygmomanometer becomes no longer usable.

Such air bag for cuff, when employed in a sphygmomanometer, is used daily and tens of thousands of times within a short period, and an improvement has therefore been desired in the durability of the cuff air bag for the sphygmomanometer, in consideration of the foregoing situation.

In order to resolve the drawbacks in the durability of the prior product, there is proposed an air bag prepared by steps disclosed in JP-A-5-64631.

In the preparation of such air bag, at first, in a rectangular resin sheet, edge portions of two arbitrary opposed sides are mutually superposed in a link-front joint or a form of joined palms as if in a prayer, namely in such a manner that the edge portions of the two sides are mutually contacted at an internal side thereof.

Thereafter, the superposed portions are welded for example with a high frequency welder to form the resin sheet into a tubular member, and such tubular member of the resin sheet is so flattened from above that thus jointed portion is positioned at an approximate center of a surface.

Then, in the resin sheet formed into such flattened planar rectangular shape, two sides constituting apertures on both ends are respectively welded to form a cuff air bag, sealed at the ends.

However, in such prior air bag, when used as a cuff air bag for the sphygmomanometer, the air brown into the cuff air bag is compressed to exert, as explained above, a force acting from the interior of the bag to the exterior, whereby each welded end of the resin sheet is repeatedly subjected to a large pressure directed from the interior of the bag to the exterior.

As a result, in such prior cuff air bag, in case the jointed portion is subjected to the aforementioned pressure, a stress pulling the air bag in a direction along the plane of the bag occurs in any position in the air bag.

In such situation, in the jointed portion formed by the link-front jointing, the face jointed by welding is approximately perpendicular to the acting direction of such stress. When the jointed portion is constantly subjected to a force in a direction for peeling off such jointed portion, since the portion jointed by welding does not have a very high adhesion strength because of a limited width thereof in the acting direction of the stress, the jointed portion shows a gradual peeling in time from a lower end thereof, thereby resulting in a leakage of the compressed air to the exterior and becoming unusable as the cuff air bag.

In consideration of the foregoing, an object of the present invention is to provide an air bag capable of maintaining satisfactory durability over a prolonged period even when used daily.

Another object of the present invention is to provide an air bag for cuff of a sphygmomanometer, capable of maintaining satisfactory durability over a prolonged period even when used daily.

### SUMMARY OF THE INVENTION

In order to attain the aforementioned objectives, the air bag of the present invention includes a step of jointing, in a sheet formed in a rectangular shape, end portions of a pair of two opposed sides so as to form a jointed portion of a predetermined width along the planar direction of the sheet.

In the present invention, therefore, the end portions of a pair of two mutually opposed sides in a rectangular sheet are jointed by a jointing portion formed along the planar direction of the sheet.

As a result, such jointing portion, being formed with a predetermined width along the planar direction of the sheet, can easily withstand a tensile stress acting in the planar direction of the sheet.

Therefore, even in case the air of a predetermined pressure is sealed inside to exert a strong force repeatedly from the interior to the exterior, the jointed portion can withstand, without showing a peeling, daily use over a prolonged period.

Also the present invention includes a first step of superposing an external surface of one of the end portions of the aforementioned sheet with an internal surface of the other end portion and jointing such superposed portions, and a second step of jointing the two other opposed sides of the remaining pair.

Thus, in the invention, the air bag of the invention can be formed by the two steps mentioned above.

Also the present invention includes a first step of superposing end portions of a pair of two opposed sides in a resin sheet formed in a rectangular shape, in such a manner that an external surface of one of the end portions is in contact with an internal surface of the other end portion and welding such superposed portions, and a second step of welding the two other opposed sides of the remaining pair.

Therefore, the air bag of the invention is formed by a resinous sheet.

Also in the second step of the invention, the portion jointed in the first step is placed in intermediate positions in the mutually opposed two other sides present in a pair and then such two other sides are welded.

In the present invention, therefore, since the aforementioned jointed portion is placed in intermediate positions in the longitudinal direction of the mutually opposed two other sides present in a pair and such two other sides are then welded, the completed air bag has the jointed portion formed in the first step in the intermediate positions in the longitudinal direction of the other two sides. Thus, when the air bag is used with compressed air therein, the tensile stress in the planar direction acts substantially uniformly on the jointed portion, whereby such jointed portion can be prevented from a situation where only one side thereof is subjected to a large stress and the jointed portion scarcely shows peeling even after use over a prolonged period. As a result, the durability of the air bag can be further improved.

Also in the invention, a resinous sheet is formed into a substantially tubular member by welding the jointing portion in the first step, and such tubular member is folded flat and remaining apertures at the ends are welded.

As a result, the invention allows to produce an air bag easily and promptly, in comparison with the prior case in which the sides of two sheets are respectively jointed by fusion.

In the invention, the aforementioned air bag is an air bag for cuff of a sphygmomanometer. Therefore a highly durable air bag for sphygmomanometer can be obtained.

In the invention, the end portions of the aforementioned two sides are welded with a welder.

### BRIEF DESCRIPTION OF THE DRAWINGS

Fig. 1 is a perspective view showing, in an embodiment of an air bag of the present invention, a resin sheet to be used for preparing an air bag.
Fig. 2 is a perspective view showing, in an embodiment of the air bag of the invention, a tubular member obtained by superposing end portions of mutually opposed two sides of the resin sheet and welding such superposed portion.
Fig. 3 is a perspective view showing a state in which the tubular member of the resin sheet is flattened into a resin sheet member of a rectangular shape.
Fig. 4 is a perspective view showing a state in which an air bag of the invention is prepared as an air bag for a cuff of a sphygmomanometer.

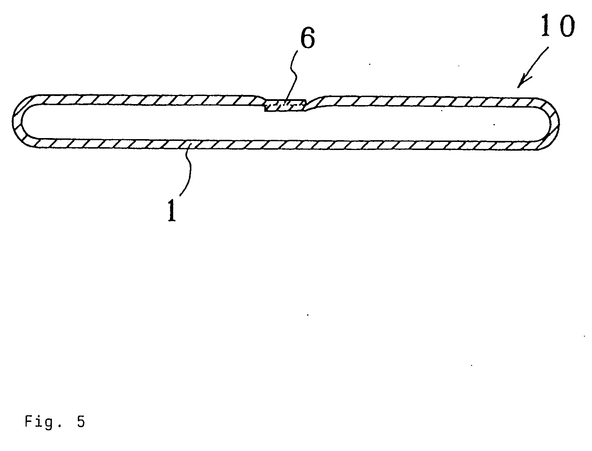

Fig. 5 is a cross-sectional view along a line A-A in Fig. 4.

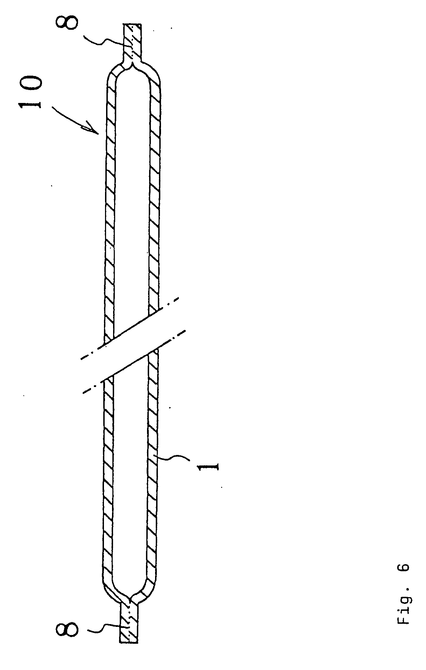

Fig. 6 is a cross-sectional view along a line B-B in Fig. 4.

### DETAILED DESCRIPTION OF PREFERRED EMBODIMENT

In the following, a process for producing an air bag for a cuff of a sphygmomanometer will be explained in detail, with reference to accompanying drawings, as an embodiment of the air bag of the present invention applied to such cuff air bag of the sphygmomanometer.

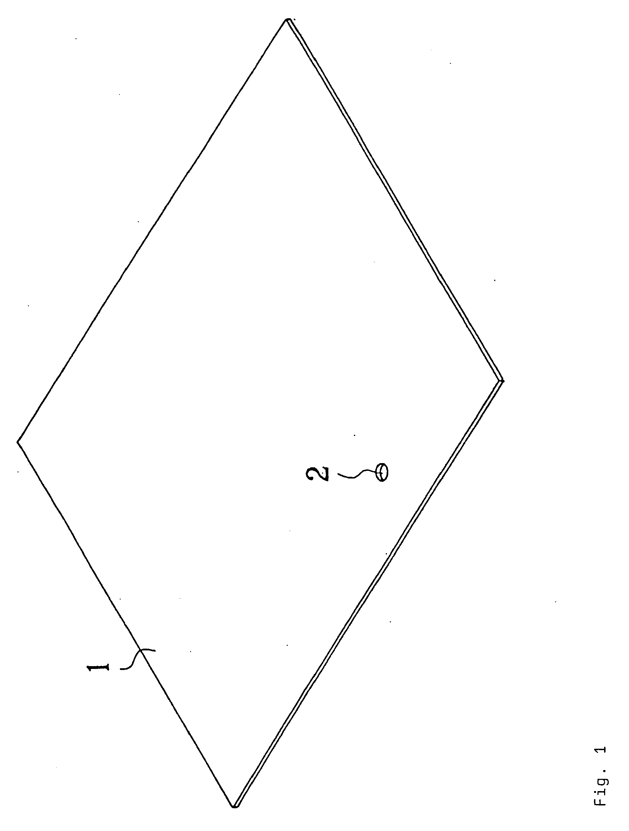

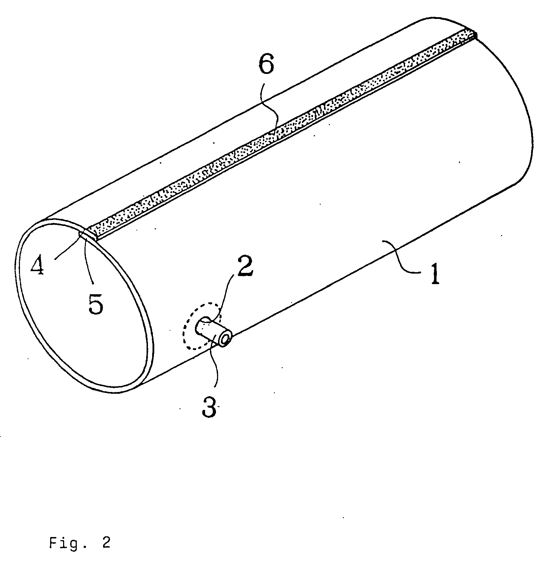

At first, as shown in Fig. 1, a PVC (polyvinyl chloride) sheet 1 of a rectangular shape is prepared, and a mounting hole 2 for mounting an air tube connector (cf. Fig. 2) is formed in a predetermined position.

Then, after an air tube connector 3 is mounted for example by welding on the mounting hole 2 of the PVC sheet 1, edge portions 4, 5 of two opposed sides of the resin sheet 1 are superposed and jointed in such a manner that an external surface of an edge portion 5 comes into contact with an internal surface of the other edge portion 4, and such jointed portion is welded by a high frequency welder to form the PVC sheet into a tubular shape. In the drawings, a numeral 6 indicates the jointed portion by welding.

The jointed portion 6 is formed with a predetermined width, and is positioned along the planar direction of the PVC sheet 1. This is different from a situation where edge portions of a PVC sheet are jointed in a prior "link-front" state to form a jointed portion substantially perpendicular to the planar direction of the sheet.

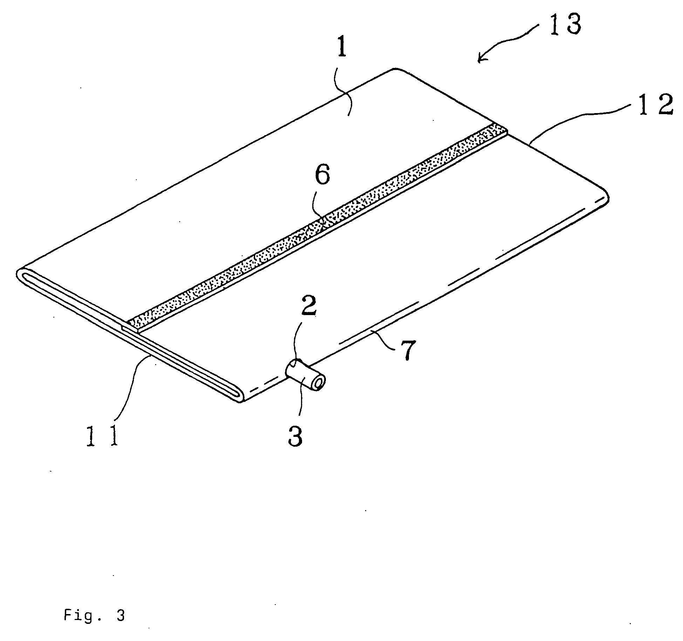

Then the tubular member of the PVC sheet 1 is flattened as shown in Fig. 3 to form a resin sheet member 13 of a rectangular shape, and the jointed portion is positioned at intermediate positions of other edge portions 11, 12 of the PVC sheet member 13.

A position of the mounting hole 2, namely a position for mounting the air tube connector 3 is preferably on a folded end 7 as shown in Fig. 3, formed when the tubular member of the PVC sheet 1 is flattened.

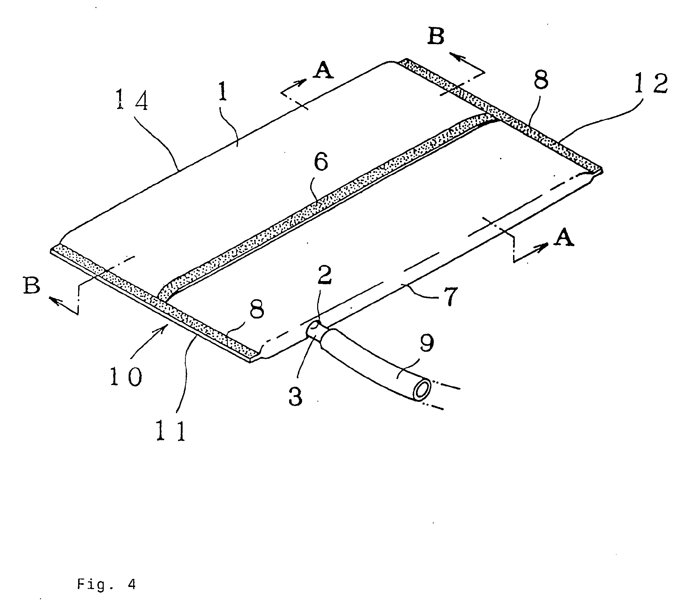

As described above, the PVC sheet member 13 of the rectangular shape is still open at both ends 11, 12 in the longitudinal direction. Therefore, as shown in Fig. 4, the both open ends 11, 12 are closed by welding for example with a high frequency welder. Then an air tube 9 is jointed to the connector 3 to complete an air bag 10 for a cuff of a sphygmomanometer.

The thus obtained cuff air bag 10 of the sphygmomanometer is formed, in the PVC sheet 1 of rectangular shape, by superposing the edge portions 4, 5 of the mutually opposed two sides in such a manner that the internal surface of the edge portion 4 comes in contact with the external surface of the other edge portion 5 and executing welding in this state with the welder, so that the jointed portion 6 formed by the welding is formed with a predetermined width, and is present over the entire width of the PVC sheet 11 and along the planar direction thereof.

The cuff air bag 10 of the sphygmomanometer of the present embodiment has an ordinary size with a transversal size of 120 mm and a longitudinal size of 240 mm, and the jointed portion 6 is formed with a width within a range of 5 to 10 mm.

Therefore, when the cuff air bag 10 of the sphygmomanometer of the present embodiment is used in a state shown in Fig. 4 and air is blown into the cuff air bag 10 and compressed therein, an air pressure is generated from the interior of the air bag to the exterior to generate a tensile stress functioning in mutually opposite directions in any position along the surface of the sheet material constituting the cuff air bag 10, and such stress acts also on the aforementioned jointed portion 6, but such jointed portion, being formed with a predetermined width along the planar direction of the sheet, can easily resist such tensile stress along the planar direction of the sheet.

In a comparison with the prior cuff air bag for the sphygmomanometer in which the welded jointing is formed in the "link-front" shape, the prior jointed portion is formed substantially perpendicularly to the acting direction of the tensile stress generated in the sheet, so that the welded joint portion has a very small width along the surface and the joint portion may show peeling, leading to an air leak from the interior when it is frequently subjected to such tensile stress in the daily use.

In the aforementioned embodiment, the jointed portion 6, being formed with a predetermined with along the planar direction of the sheet, can sufficiently resist the tensile stress applied along the planar direction of the sheet and does not show peeling, whereby the durability can be significantly improved in comparison with the prior cuff air bag for the sphygmomanometer.

Also in the present embodiment, the jointed portion 6 is provided in intermediate positions of the mutually opposed two sides 7, 14 along the transversal direction of the air bag 10 and such other two sides are welded in such state, so that, when the air bag is used with compressed air therein, the tensile stress in the planar direction is applied constantly and substantially uniformly on the jointed portion 6. Thus there can be avoided a situation where a large stress acts only on either side of the jointed portion 6, which is hardly peeled off even after a prolonged use. As a result, the durability of the cuff air bag 10 for the sphygmomanometer can be improved further.

Also in the invention, the resin sheet is formed, by the welding of the jointing portion in the first step, into a substantially tubular member, then such tubular member is folded substantially flat and the remaining open ends are welded.

The cuff air bag 10 for the sphygmomanometer of the present embodiment has been explained by a case utilizing welding with a welder, but such embodiment is not restrictive and any jointing method for fused jointing of resin such as an ultrasonic welding method or a heat sealing method.

Also the present invention has been explained by an embodiment in which the air bag of the present invention is applied to an air bag for a cuff of a sphygmomanometer, but such embodiment is not restrictive and the present invention is applicable to various other applications such as air bags for various inspections and air bags for massaging.

Also the present embodiment has been explained by a cuff air bag of an ordinary size, but such embodiment is not restrictive. For example a small cuff air bag has a transversal dimension of 60 to 65 mm, a longitudinal dimension of 100 to 125 mm, and a width of the jointed portion of 3 to 5 mm, while a large cuff air bag has a transversal dimension of 160 mm, a longitudinal dimension of 330 mm, and a width of the jointed portion of 10 mm.

In the cuff air bag of any size, a test for peeling strength was conducted on the jointed portion and proved a value close to a destruction strength of the PVC sheet itself.

## Claims

1. A method for producing an air bag, comprising a step of jointing, in a sheet formed in a rectangular shape, end portions of a pair of mutually opposed two sides in such a manner as to form a jointed portion of a predetermined width along a planar direction of the sheet.

2. A method for producing an air bag according to claim 1, comprising a first step of superposing in such a manner an external surface of an either end of said sheet comes into contact with an internal surface of the other end and jointing the superposed portion, and a second step of jointing a remaining pair of mutually opposed two sides.

3. A method for producing an air bag according to claim 2, comprising a first step of superposing, in a sheet made of resin formed in a rectangular shape, end portions of mutually opposed two side in such a manner an external surface of an either end portion comes into contact with an internal surface of the other end portion and welding thus superposed portions, and a second step of welding remaining a remaining pair of mutually opposed two sides.

4. A method for producing an air bag according to claim 3, wherein, in said second step, the remaining two sides are welded with the jointed portion, formed in the first step, being placed in intermediate positions of the remaining pair of mutually opposed two sides.

5. A method for producing an air bag according to claim 4, wherein the resin sheet is formed by welding said jointing portion in said first step into a substantially tubular member, and said tubular member is folded in a substantially flat shape and remaining open ends are welded.

6. A method for producing an air bag according to claim 5, wherein said air bag is an air bag for a cuff of a sphygmomanometer.

7. A method for producing an air bag according to claim 6, wherein the end portions of said two sides are respectively welded by a welder.

8. A method for producing an air bag according to claim 7, wherein said resin sheet is a PVC sheet.
